# EUROPEAN PATENT APPLICATION

(11) **EP 4 811 381 A1**
(43) Date of publication of application: **23.09.2026**
(21) Application number: 25164715.2
(22) Date of filing: 19.03.2025
(51) Int. Cl.: G16H 30/20, G16H 30/40

(54) **TECHNIQUE FOR MEDICAL IMAGING ANALYSIS IN RELATION TO A MEDICAL QUESTION**

(71) Applicant: Siemens Healthineers AG, 91301 Forchheim (DE)
(72) Inventor: MANSOOR, Awais, Potomac, 20854 (US); GEORGESCU, Bogdan, Princeton, 08540 (US); SCHMUECKING, Ingo, Yardley, 19067 (US); GRBIC, Sasa, Plainsboro, 08536 (US); MITSCHKE, Matthias, 90491 Nürnberg (DE); HÜMMER, Christian, 96215 Lichtenfels (DE); SAHU, Pranjal, Morrisville, 19067 (US)
(74) Representative: Schwarz, Claudia

(57) **Abstract**

The invention relates to a technique for extracting medical imaging analysis data in relation to a medical question. A method (100) comprises a step of receiving (S102) a first medical dataset in relation to a patient. The first medical dataset comprises first medical imaging data acquired by means of a first medical imaging modality in relation to a medical question. A second medical dataset in relation to the patient is received (S104). The second medical dataset comprises context information in relation to the medical question. At least part of the received (S102) first medical dataset is converted (S106) into a target format. The at least in part converted (S106) first medical dataset and the received (S104) second medical dataset are jointly analysed (S108) in view of the medical question. Medical imaging analysis data in relation to the medical question are extracted (S110) from the result of the jointly analysing (S108).

## Description

The present invention relates to a technique for extracting medical imaging analysis data in relation to a medical question, in particular comprising a method, a computing device, a unified platform, and a computer program product.

Using prior images and data with the currently acquired imaging is paramount to radiology reading and to producing meaningful diagnosis, and thus to the radiology report. Given the dire shortage of radiologists across the globe, various efforts have been presented on the topic of autonomous reading of radiology images. The effort has recently also seen much more openness from the clinical community than before. Although current Al systems have demonstrated state-of-the-art performance, outperforming expert radiologists in certain instances and tasks, the translation of these systems into clinical routine is still limited, which is problematic in particular given the lack of radiologists, the workload thus creating a huge demand for autonomous or semi-autonomous systems. However, these systems may have the drawback that they do not sufficiently and extensively consider the context of the information. Contrarily to conventional automated data processing, radiologists and other clinical experts are capable of putting the current imaging findings in the context of (1) non-imaging data (patient history, EHR, demographic information, etc.), and (2) prior-imaging.

The problem of comparing multiple exams is challenging for conventional Al systems primarily due to the fact that these systems suffer from performance degradation due to large shifts of data distributions. This is compounded by the multimodal nature, such as prior exams performed in another imaging modality, which is a common occurrence in radiology workflow. The multimodal nature of clinical exams over a patient's history often includes different imaging modalities. Moreover, sometimes the prior exam is present only in the shape of a clinical report without any imaging.

Image homogenization techniques have been proposed in the literature with moderate success, which were primarily borrowed from the computer vision community. However, prior information sometimes come in the shape of a mere clinical report without imaging. Al systems conventionally struggle to effectively handle the diverse array of information sources, something that is extremely easy for human expert.

It is therefore an object of the present invention to provide a solution for (in particular at least semi-automatically) jointly analysing a patient's health-related information in its context, for example over the patient's history. Alternatively or in addition, a task is to facilitate extracting, combining, and/or putting into context health-related data. The data may stem e.g. from longitudinal and/or multi-modality studies, such as imaging data from various medical imaging modalities and/or text data. Alternatively or in addition, it is an open problem to support a medical practitioner in improved diagnosis and/or treatment planning based on the collective patient's medical history.

This object is solved by a method for extracting medical imaging analysis data in relation to a medical question, by a computing device, by a unified platform, and by a computer program (and/or a computer program product) according to the appended independent claims. Advantageous aspects, features and embodiments are described in the dependent claims and in the following description together with advantages.

In the following, the solution according to the invention is described with respect to the claimed method as well as with respect to the claimed computing device Features, advantages or alternative embodiments, mentioned with respect to the method can be assigned to the other claimed objects (e.g. the computer program or a device, in particular the unified platform, , the computing device, or computer program product) and vice versa. In other words, the system, apparatus or device can be improved with features described or claimed in the context of the method and vice versa. In this case, the functional features of the method are embodied by structural units of the apparatus or device or system and vice versa, respectively. The method may refer to a software implementation and the device may refer to a hardware implementation (with a spatial physical structure). Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the respective device or apparatus claim is configured to carry out the claimed method.

As to a method aspect, a (in particular computer-implemented) method for extracting medical imaging analysis data in relation to a medical question is provided. The method comprises a step of receiving a first medical dataset in relation to a patient. The first medical dataset comprises first medical imaging data acquired by means of a first medical imaging modality in relation to a medical question. The method further comprises a step of receiving a second medical dataset in relation to a (e.g., the same or a similar, such as for comparative purposes) patient. The second medical dataset comprises context information in relation to the medical question. The method further comprises a step of converting at least part of the received first medical dataset into a target format (and/or a format in that both first and second medical dataset can be processed, such as a shared space). The method further comprises a step of jointly analysing the at least in part converted first medical dataset and the received second medical dataset in view of the medical question. The method still further comprises a step of extracting medical imaging analysis data in relation to the medical question from the result of the jointly analysing.

By the inventive technique, and accuracy and/or reliability of medical image analysis data (and/or health-related information of a patient; also: proposal medical features in relation to the patient), such as an existence and/or type of anomaly, extracted from two or more medical datasets can be improved. Alternatively or in addition, a performance degradation can be avoided that conventionally occurs, e.g., due to large shifts of data distributions.

By the inventive technique, an automated reading of medical imaging data and/or medical non-imaging data can be enabled, which can comprise historic (and/or previously acquired, and/or longitudinal) medical data and/or current (and/or more recently acquired) medical data. Thereby, e.g., as an image analysis result a change in the patient's health state may be detected and/or monitored (e.g., the progression of an anomaly, such as the growing of a tumor or the shrinking of a lesion).

The extracting of the medical image analysis data from the first medical dataset can be improved by means of the context information (also: context data, and/or context knowledge) comprised in the second medical dataset.

The first medical dataset comprises first medical imaging data, in particular acquired by a first medical scanner, e.g., using magnetic resonance tomography (MRT), computed tomography (CT), ultrasound (US), positron emission tomography (PET), X-ray imaging, and/or others. The second medical dataset may comprise second medical imaging data, in particular acquired by a second medical scanner, which may also be of different modality type with respect to the first scanner. The first medical scanner and the second medical scanner may be independent or different, such as in terms of their medical imaging modality and/or in terms of their technical specifications (e.g., comprising different magnetic field strengths for two different MRT scanners, and/or single vs. dual energy CT scanners). The first medical dataset (and optionally the second medical dataset) may comprise preliminary medical image analysis data and/or a result of a perception task. The perception task may comprise detection (e.g., organ detection and/or object detection, such as detection of a stent or other implant) and/or segmentation (e.g., semantic segmentation).

Any (e.g., the first and/or second) medical imaging data may comprise raw data acquired by means of a medical scanner of the corresponding medical imaging modality. Alternatively or in addition, any (e.g., the first and/or second) medical imaging data may comprise pre-processed and/or reconstructed imaging data acquired by means of the medical scanner.

Alternatively or in addition, the first medical dataset and/or the second medical dataset may comprise text data (also: clinical data, and/or clinical report), such as a medical examination report (briefly: medical report; also: clinical report), annotations on the respective medical imaging, laboratory results, and/or (prescribed and/or non-prescribed) medications taken by the patient.

The context information may be related to medical questions for the patient, and in particular to the same medical question for which the first medical dataset was acquired. Alternatively or in addition, the context information may be patient-specific. The context information may comprise the second medical imaging data and/or the text data (also: non-imaging data). The second medical imaging data may, e.g., comprise prior studies in relation to the same patient and/or may be acquired by means of a second medical imaging modality, which may be the same as, or different from, the first medical imaging modality. Alternatively or in addition, the text data comprised in the second medical dataset may comprise an electronic health record (EHR), a medical report, examination results and/or laboratory results in text form (also: textual format), and/or the patient's medical history for the respective patient, i.e. the subject of the first medical dataset.

The context information may comprise results of an artificial intelligence (AI) tool, e.g., of a large language model (LLM) for the text data. Alternatively or in addition, one or more LLMs can be used to extract information from non-imaging sources (and/or textual formats, and/or text data) that may be relevant for clinical diagnosis.

The context information may alternatively or in addition comprise data which are output(s) of one of a plurality of image analysis algorithms. For example, in an embodiment, a prior study, in particular for the same medical question (in particular of the same patient for whom the first medical imaging data are received), is to be considered. The prior study may have already been processed by means of at least one image analysis algorithm, e.g. an Al tool (such as for detection and/or segmentation). The output of this Al tool, preferably already provided in the target format, e.g., textual format, may be provided as context information to be processed jointy (and/or in combination) with the received first medical dataset. Otherwise, if the output of the image analysis algorithm of the second medical dataset is not provided in the target format, a second conversion may be applied to transfer the same in the target format, so that the first medical dataset and the second medical dataset may be processed jointly or in common. In an example, the target format may be a textual format, which enables the use and application of LLMs. Alternatively or in addition, the target format may comprise a segmentation (e.g., for a specific organ according to the medical question).

The first and second medical dataset may be related to the same patient. Alternatively or in addition, the first and second medical dataset may be related to two different patients, such as for inter-cohort and/or intra-cohort comparison of characteristics (e.g., related to a specific type of lesion).

The first and second medical dataset may be acquired at different instances in time. E.g., the first medical dataset may comprise more recent medical data than the second medical dataset.

Any of the medical datasets may provide context, e.g., in terms of text data. Alternatively or in addition, the first and second medical dataset may be acquired by means of different medical scanners, such as medical scanners of different modalities.

The first medical dataset may be received in a source format. Alternatively or in addition, the second medical dataset may be received in the target format.

The technique may be modality agnostic and/or may be suitable for multi-modality extracting of medical imaging analysis data and/or for extracting of health-related information. E.g., by converting the received first medical dataset into the target format, different types of medical imaging data may be directly compared. For example, a three-dimensional (3D) medical image may be compared with a two-dimensional (2D) medical image, such as by selecting (and/or constructing) a corresponding 2D projection (also: view).

In some embodiments, the target format may be a format that both, the first medical dataset and the second medical dataset, can at least partly be converted into, and/or a format in that both, the first medical dataset and the second medical dataset, can be processed or understand. Alternatively or in addition, converting into a target format may involve using a shared space (e.g., shared between the first medical dataset and the second medical dataset).

Converting at least part of the first medical dataset into the target format may comprise applying an imaging Al (Al stands as abbreviation for artificial intelligence, e.g., embodied by a neural network system, such as a CNN, U-Net, and/or Transformer) and/or a perception task model, and/or image processing tools. The imaging AI, perception task model, and/or image processing tools may be configured for specific medical tasks, comprising organ detection (and/or object detection), and/or for (in particular semantic) segmentation. Alternatively or in addition, the imaging AI, perception task model, and/or image processing tools may be specific to the medical imaging modality. Any (in particular modality-specific) imaging Al may be an Artificial Narrow Intelligence (ANI).

Alternatively or in addition, converting the first medical dataset into the target format may comprise applying a LLM on text data.

Alternatively or in addition, converting at least part of the first medical dataset into the target format may comprise converting at least part of the first medical imaging data (e.g., from a source format) into the target format.

According to some examples, the step of converting at least part of the first medical dataset into the target format may comprise determining the target format based on the second medical data set, optionally, a source format of the second medical data set, based on the clinical question, and/or based on the step of jointly analysing, optionally, a foundational model and/or an analysis algorithm used in the step of jointly analysing, further optionally, formal requirements regarding the input data of the foundational model and/or analysis algorithm used in the step of jointly analysing. With that, the target format can be identified according to the requirements of the further processing which improves compatibility and efficiency.

Applying a LLM on text data may comprise transforming the text data according to a predetermined medical ontology, and/or converting the text data into a predetermined medical terminology.

Jointly analysing the converted first medical dataset and the second medical dataset may comprise jointly analysing at least part of the first medical imaging data and the context information.

Jointly analysing the converted first medical dataset and the second medical dataset may be performed by means of a foundational model and/or by means of an analysis algorithm.

The LLM, the analysis algorithm, the foundation model, and/or a perception task model may be trained, in particular by means of supervised learning using training pairs of medical datasets annotated by a medical expert. Alternatively or in addition, the LLM, the analysis algorithm, the foundation model, and/or the perception task model may be fine-tuned when performing the method, e.g., based on acceptances, rejections, and/or rating of the extracted medical imaging analysis data by a medical expert.

Alternatively or in addition, training the LLM, the analysis algorithm, the foundation model, and/or the perception task model may make use of self-supervised learning (SSL) and/or (in particular dual-branch) self-distillation.

The foundation model may be configured for jointly analysing text data and/or medical imaging data. Alternatively or in addition, the foundation model may be configured for determining medical data comprised in both, the first and second, medical dataset, which may be jointly analysed. Alternatively or in addition, the foundation model may be configured to extract the medical question from the first medical dataset, and for scrutinizing the second medical dataset, or both medical datasets, in view of information relating to the medical question. E.g., an indication of the medical question may be extracted from the metadata of the (e.g., first) medical dataset. Alternatively or in addition, an indication of the medical question may be extracted from a medical imaging protocol used for the acquisition of the medical imaging data.

Jointly analysing the medical datasets may comprise selecting a subset of medical data comprised in each individual medical dataset, and focusing on the medical data in both medical datasets that may be associated with the medical question. E.g., at least one of the first and/or second medical dataset may comprise medical imaging data of a patient's thorax, but the medical question may only relate to one of the imaged organs, e.g., only the lungs.

The extracted medical imaging analysis data (and/or health-related information) may comprise a newly detected anomaly and/or a progression of an anomaly (e.g., an aggravation or an improvement of a health state).

According to an embodiment, the (e.g., first and/or second) medical imaging data may be received as obtained from a scanner and an image analysis may be performed upon reception, e.g., by using Al tools, such as for converting at least part of the first medical dataset into the target format. In a further embodiment, which is combinable (e.g., for further first and/or second medical imaging data) with the previous embodiment, already processed (e.g., first and/or second) and/or converted medical imaging data may be received, and only the joint analysing is performed by the inventive technique.

A purpose of the inventive technique (e.g., comprising a unified platform; also: mutually understandable space, briefly: space) is to provide a unified framework that can be understood across the board, e.g., to make comparisons. In one embodiment, for instance, the first medical dataset (also: source data) and the second medical dataset (also: target data) reside in two separate jurisdictions, and it may not be possible to share the actual (in particular, source and/or target) data due to privacy concerns. In that embodiment, the Al tools may run locally (e.g., for the second medical dataset) and populate the fields of this mutually understandable space (also: unified platform). The populated fields of this space may then be communicated to the other site for comparison (e.g., with the first medical dataset, or vice versa in terms of first and second) instead of the actual (in particular target and/or source) data. In another embodiment, the data (e.g., the first medical dataset, or at least the first medical imaging data, and/or the second medical dataset, or at least the context information) may be received and then the image analysis may be performed using Al tools.

In an embodiment, medical imaging information and contextual information can be used jointly to train a computing device or a distributed computing system, and/or a computing cloud, such as according to the technique provided in [4], for the jointly analysing of the first and second medical dataset (and/or the first medical imaging data and the context information comprised therein, respectively).

The inventive technique may improve over the prior art in terms of computational speed, and/or a reduced need for computing resources, in particular processing resources and/or memory space. Alternatively or in addition, the extracted medical imaging analysis data may be improved, e.g., in terms of smaller error bars on the estimated size and/or shape of a lesion.

The first (and/or the second) medical dataset may comprise metadata (e.g., in a predetermined format), e.g., the patient's personal data, such as name, birth date, gender, height, potential amputations, and/or potential implants; and/or date and/or type of examination of the patient, such as imaging and/or non-imaging.

Alternatively or in addition, the text data may be provided according to a predetermined medical ontology. Optionally, the text data may comprise, in relation to the patient, his or her medical history; a medical condition, in particular as previously reported and/or derived from an examination; a medication, such as prescribed and/or non-prescribed; and/or textual annotation of medical imaging data (e.g., a measured and/or estimated size of a lesion).

Annotations of medical imaging data may be provided in terms of predetermined organ categories and/or object categories (e.g., for implants, such as a stent, pacemaker, dental implant, hip implant, knee implant, and/or any other type of implant); and/or in terms of predetermined (e.g., semantic and/or organ) segmentation categories.

The metadata may comprise administrative data that identify the patient and/or that ensure that only medical datasets pertaining to the same patient are compared. Alternatively or in addition, the metadata can serve to order the medical datasets chronologically, and/or to determine the target format (and/or any standardized format) to be used for the joint analysing (and/or the comparing for the first and second medical datasets). E.g., the metadata may comprise an indication if medical imaging data comprised in the medical dataset are 3D (also: volumetric) or 2D (also: planar). Alternatively or in addition, the metadata may comprise an indication of a resolution of the medical imaging data.

In an alternative embodiment, the metadata may comprise data for selecting the second medical dataset (and/or a second patient) for jointly analysing with the first medical dataset (and/or the patient), such as based on the medical question, e.g., in case of rare diseases and/or rare cancers, such as for comparing with a previous (and/or the second) patient treated for the same rare disease and/or rare cancer.

Jointly analysing the medical data of the first and second medical datasets may comprise comparing the medical data at a selected dimensionality (in particular 2D if one of two medical imaging datasets is 2D and the other 3D) and/or a selected resolution (e.g., the coarser granularity among two medical imaging datasets).

The received first medical dataset, or the converted first medical dataset, may comprise a result of a perception task. Optionally, the perception task may comprise an organ detection and/or a semantic segmentation.

The perception task may be performed by (and/or the result of the perception task may be obtained from) a perception task model. The perception task model may comprise a detection model and/or a segmentation model. The perception task may be performed on the first (and/or second) medical imaging data.

The segmentation model may be configured for performing semantic segmentation, in particular according to a predetermined set of segmentation classes. Alternatively or in addition, the detection model may be configured for performing organ detection and/or object detection (e.g., for detecting implants), in particular according to a predetermined set of object classes.

The converting of at least part of the first medical dataset may depend on the received context information and/or on the medical question.

The context information, the medical question, and/or any text data may be analysed by means of an LLM.

The received second medical dataset may comprises text data. Optionally, the text data may comprise a medical report of a medical examination, the patient's medical history, the patient's medication, laboratory result data; and/or annotations in relation to medical imaging data acquired by means of a second medical imaging modality.

Alternatively or in addition, in addition to the first medical imaging data, the first medical dataset may comprise annotations (e.g., a segmentation result, a detection result, and/or a result of a measurement and/or an estimate of a lesion or organ, e.g., a length or diameter) to the first medical imaging data.

The received second medical dataset may comprise second medical imaging data acquired by means of a second medical imaging modality, in particular in relation to the medical question.

The second medical imaging data may be acquired before the first medical imaging data. Alternatively or in addition, the second medical imaging data may have a lower dimension (e.g., 2D) and/or a lower resolution than the first medical imaging data (which may, e.g., be 3D).

The first medical imaging modality (and optionally the second medical imaging modality) may comprise MRT, CT, single-photon emission CT (SPECT), PET, scintigraphy, radiography (also: X-ray imaging, XR), ultrasound (US), elastography, photoacoustic imaging, functional near-infrared spectroscopy (FNIR), and/or magnetic particle imaging (MPI). In some embodiments, the first medical imaging modality (and optionally the second medical imaging modality) may comprise a combination of two medical imaging modalities, such as PET-CT.

The (e.g., first and/or second) medical imaging data may comprise 3D data (also: volumetric data and/or a volumetric image; e.g., acquired by means of MRT and/or CT) or 2D data (also: planar data and/or a planar image; e.g., acquired by means of radiography; also: X-ray imaging).

Converting the first medical imaging data into the target format may be determined on a case-by-case basis, in particular depending on the second medical dataset comprising the second medical imaging data to be used for the jointly analysing, and/or depending on the medical question. In some embodiments, in particular volumetric medical imaging data, may be useful for answering different medical questions, and the target format may be chosen according to a selected one among these medical questions.

The second medical imaging modality may differ from the first medical imaging modality. The second medical imaging data may have been acquired prior to the first medical imaging data.

By acquiring medical imaging data using different medical imaging modalities, use may be made of the most recent technological developments, and/or or the availability of a specific scanner, when acquiring the more recent (in particular the first) among the two medical datasets. Alternatively or in addition, different medical imaging modalities may be optimal for detecting different types of anomalies. By combining medical imaging data from two different medical imaging modalities, a detection rate and/or a reliability of detecting an anomaly may be improved. Alternatively or in addition, different types of information (e.g., complementary information, which may aid in distinguishing between different diagnoses) about the detected anomaly may be obtained using the different medical imaging modalities.

The second medical imaging data may be lower dimensional than the first medical imaging data. In particular, the second medical imaging data may comprise a 2D medical image, and the first medical imaging data may comprise a 3D medical image. Alternatively or in addition, converting at least part of the first medical dataset into the target format may comprise constructing a 2D medical image from the first medical imaging data, in particular comprising a similar view on anatomical structures as a 2D medical image comprised in the second medical dataset.

Converting at least part of the first medical dataset into the target format may comprise determining the dimensionality of the second medical imaging data and/or determining a view (e.g., a viewing direction and/or a position of at least one point on a 2D image, and/or a corresponding slice in the 3D medical image to the 2D image) comprised in the second medical imaging data.

The similarity of the constructed 2D medical image from the first medical imaging data to the 2D medical image comprised in the second medical imaging data may provide similar anatomical structures for rendering and/or similar directions for performing measurements, e.g., of lesions. Thereby, a development of a medical condition (and/or of a lesion) may be monitored with improved accuracy and/or without requiring time-consuming manual selections of suitable views of the first medical imaging data.

The method may at least partly be performed by a target-perceived dual-branch distillation (TDD) network. The TDD network may comprise a digital reconstructor. The digital reconstructor may be configured for converting the first medical imaging data and for obtaining a similar view to a view provided by the second medical imaging data. The digital reconstructor may further comprise a proposal extractor. The proposal extractor may be configured for identifying at least one region of interest (ROI) in the converted first medical imaging data and in the second medical imaging data. The digital reconstructor may further comprise a source-adaptive (SA) block and a target-like (TL) block. The SA block may be configured for performing medical image data analysis on the first medical imaging data, in particular as received and/or before converting to the target format. The TL block may be configured for performing medical image data analysis on the converted first medical imaging data, in particular on at least part of the first medical imaging data converted into the target format. Alternatively or in addition, the TL block may be configured for performing medical image data analysis on at least part of second medical imaging data comprised in the second medical dataset. The digital reconstructor may still further comprise a target proposal perceiver. The target proposal perceiver may be configured for comparing the medical image data analysis from the SA block and from the TL block.

The proposal extractor may comprise a region proposal network (RPN) for identifying ROIs.

The SA block and/or the TL block may comprise one or more fully connected (FC) layers and/or a projection head (HEAD).

The target proposal perceiver may perform iterative cross-attention among layers of the SA block and layers of the TL block. The target proposal perceiver may in particular be used for training the SA block and the TL block jointly, for example based at least partly on annotated (in particular first) medical imaging data, and/or at least partly on dual-branch self-distillation. Thereby, domain knowledge from the source domain (and/or of data in the source format) may be used in the target domain (and/or for data in the target format), and vice versa.

The TDD may be trained using supervised training (in particular ground truth knowledge), in particular for the SA block and the TL block separately, and/or self-distillation, in particular based on the results of the target proposal perceiver.

Alternatively or in addition, the SL branch and the TL branch may be trained in a unified teacher-student learning. E.g., the SL branch may take as input the first medical imaging data (in particular in the source format) along with annotations as ground truth, and/or the SL branch may act as teacher. The annotations may be transferred to the converted first medical imaging data (in particular in the target format) for use for training the TL branch, and/or the TL branch may act as student.

Dual-branch self-distillation may comprise training by (e.g., second) medical imaging data without annotation, and/or with a pseudo-annotation generated in (or by) the SL branch (e.g., as teacher). The pseudo-annotation may be used for training the TL branch (e.g., as student).

The step of jointly analyzing the at least in part converted first medical dataset and the received second medical dataset may be stopped if it is determined that the second medical dataset cannot answer the medical question associated with the first medical dataset. Alternatively or in addition, the step of extracting of the medical imaging analysis dataset may be stopped if it is determined that the second medical dataset cannot answer the medical question associated with the first medical dataset.

The second medical dataset may, e.g., be unsuitable for answering the medical question if it is determined that only context information on organs and/or medical conditions differing from those comprised in the first medical dataset can be extracted. E.g., if the second medical dataset comprises second medical imaging data of the patient's knee, but the medical question and first medical imaging data refer to the head or thorax, the method may be stopped.

A medical expert (e.g., radiologist) may be alerted to (and/or informed about) the stop of the joint medical imaging analysis. By the alert, an efficiency of the clinical workflow may be improved.

Alerting the medical expert may be performed by means of a user interface (UI), in particular a graphical user interface (GUI).

The method may further comprise a step of providing the extracted medical imaging analysis data to a medical expert, in particular by using a UI, preferably a GUI. Optionally, the method may further comprise a step of receiving feedback by the medical expert. The feedback may comprise an acceptance, a rejection, and/or a rating or assessment (e.g., regarding a quality) of the medical imaging analysis data. Alternatively or in addition, the acceptance, and/or the rating or assessment may be implicit, e.g., if the medical expert at least partly modifies the extracted medical imaging analysis data.

Based on the feedback, the medical imaging analysis data may (or may not) be used for drafting an examination report in relation to the first medical imaging data. E.g., the extracted medical imaging analysis data may be provided in a template for the examination report, in particular unless the medical expert rejected the extracted medical imaging analysis data.

Accepting, rejecting and/or rating or assessing the extracted medical imaging analysis data may be used for (e.g., continuously) fine-tuning the LLM, the analysis algorithm, the foundation model, the perception task model, and/or the TDD network, by which the method is performed.

The method may be performed by a computing device, by a distributed computing system, and/or in a computing cloud. Alternatively or in addition, the method may make use of a "plug- and-play" configuration in relation to task-specific Als. For example, the first medical dataset may comprise a (in particular volumetric) chest CT dataset as first medical imaging data. The chest CT dataset may be automatically analysed (e.g., measurements may be performed) by a dedicated Al tool (e.g., the Al-Rad Companion Chest CT as Al-powered radiology assistant). As further example, that is combinable with the previous example, the second medical dataset may comprise a (in particular planar) chest X-ray dataset as second medical imaging data. The chest X-ray dataset may be automatically analysed by a further dedicated Al tool (e.g., the Al-Rad Companion Chest X-Ray for detecting radiographic findings.

As to a further aspect, a unified platform for extracting medical imaging analysis data in relation to a medical question is provided. The unified platform is configured for storing a plurality of (in particular second) medical datasets. The unified platform is further configured to be accessed for performing the method according to the method aspect. E.g., the unified platform may comprise data storage space and at least one computing device, a distributed computing system, and/or a computing cloud.

The inventive technique can comprise a modality-agnostic (and/or unified) platform to store clinical information that once done, obliviates the need to have the actual data at hand for comparison, e.g., with another target and/or another time-point. The inventive technique can in particular be used to extract the clinical information where the two time-points, which the relevant information is coming, stem from different type of modalities (also denoted as source and target). For instance, the patient may have gone to hospital 1 and gotten a chest X-ray scan, and 6 months later may have gone to a different hospital (e.g., hospital 2) and gotten a (in particular chest) CT scan. Radiologists are perfectly capable of comparing the two, the X-ray and the CT scan, and do it all the time. But comparing the two, X-ray and CT scan is not very easy for the machines. According to the inventive technique, an Al system can fill out the machine-readable form (and/or the fields of the unified platform) at hospital 1, and then systems at hospital 2 will fill out a similar form (and/or fields of the unified platform). In the end, when it comes to comparing the two exams from different sites and different modalities (in particular just like human radiologists do), the Al is comparing the two forms. In particular, there is no need to worry about the fact that one scan was a chest X-ray and the other scan was a chest CT.

In particular, the unified platform may comprise a data storage space, in particular a database, which is capable of storing data (and/or information) from various different (e.g., medical imaging and medical non-imaging) sources, such as various medical scanners, measurements (e.g., comprising electrocardiograms, ECGs, electroencephalograms, EEGs, and/or laboratory results) and/or clinical reports (also: medical reports). Fields in the data storage space, in particular in the database, may be in text format (e.g., for medical reports and/or laboratory results), graph format (e.g., for ECGs, and/or EEGs), 2D image format (e.g., for X-ray images), and/or 3D image format (CT scans, and/or MRT scans). The database may be, or may comprise, an electronically stored, systematic collection of data. It can contain any type of data, including words, numbers, images, videos, and files. Software may be provided and used for the database, called a database management system (DBMS), to store, retrieve, and/or edit data.

Alternatively or in addition, the unified platform may comprise an Al orchestrator for orchestrating and/or managing a dataflow between external Als (such as one or more LLMs, and/or one or more imaging Als, in particular at least one imaging Al per medical imaging modality) and the database. The Al orchestrator may be embodied by at least one computing device (e.g., according to the device aspect), a distributed computing system, and/or a computing cloud.

The Al orchestrator may be trained with background knowledge from the medical domain, e.g., by means of self supervised learning (SSL), transfer learning, and/or reinforcement learning. Alternatively or in addition, the Al orchestrator may be configured for comprehending causes and effects, such as comorbidities, for detecting conflicting data, and/or for resolving conflicts in data (e.g., by assessing a confidence level per, in particular medical imaging data and/or medical non-imaging data, source).

As to a device aspect, a computing device for extracting medical imaging analysis data in relation to a medical question is provided. The computing device comprises a first interface configured for receiving a first medical dataset in relation to a patient. The first medical dataset comprises first medical imaging data acquired by means of a first medical imaging modality in relation to a medical question. The computing device further comprises a second interface configured for receiving a second medical dataset in relation to the patient. The second medical dataset comprises context information in relation to the medical question. The computing device further comprises a conversion module configured for converting at least part of the received first medical dataset into a target format. The computing device further comprises an analysis module configured for jointly analysing the at least in part converted first medical dataset and the received second medical dataset in view of the medical question. The computing device still further comprises an extraction module configured for extracting medical imaging analysis data in relation to the medical question from the result of the jointly analysing.

The computing device may comprise a providing module and/or a third interface configured for providing the extracted medical imaging analysis data to a medical expert, in particular by using a GUI. The computing device may further comprise a fourth interface configured for receiving feedback by the medical expert. The feedback may comprise an acceptance, a rejection, and/or a rating (also: assessment) of the medical imaging analysis data.

The computing device may be configured to perform any one of the steps, and/or may comprise any one of the features, disclosed in the context of the method aspect.

As to a further aspect, a computer program product is provided. The computer program product comprises program elements which induce a computing device (e.g., according to the device aspect), when the program elements are loaded into a memory of the computing device, to carry out the steps of the method for extracting medical imaging analysis data in relation to a medical question according to the method aspect.

As to a still further aspect, a computer-readable medium is provided, on which program elements are stored that can be read and executed by a computing device (e.g., according to the device aspect), in order to perform steps of the method for extracting medical imaging analysis data in relation to a medical question according to the method aspect, when the program elements are executed by the computing device.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labelled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: is a flow chart of an exemplary method for extracting medical imaging analysis data in relation to a medical question;
- Fig. 2: is an overview of the structure and architecture of an exemplary computing device for extracting medical imaging analysis data in relation to a medical question, which computing device may be configured for performing the method of Fig. 1;
- Figs. 3A and 3B: show an example of a unified platform which is populated, at different time points, by medical imaging and medical non-imaging data, which can serve as, or be comprised in, medical datasets used when performing the method of Fig. 1;
- Fig. 4: shows an example of performing the method of Fig. 1 for context information comprised in prior medical imaging data; and
- Fig. 5: shows a further example of the working of the method of Fig. 1 in connection of with the unified platform of Fig. 3A.

Any reference signs in the claims should not be construed as limiting the scope.

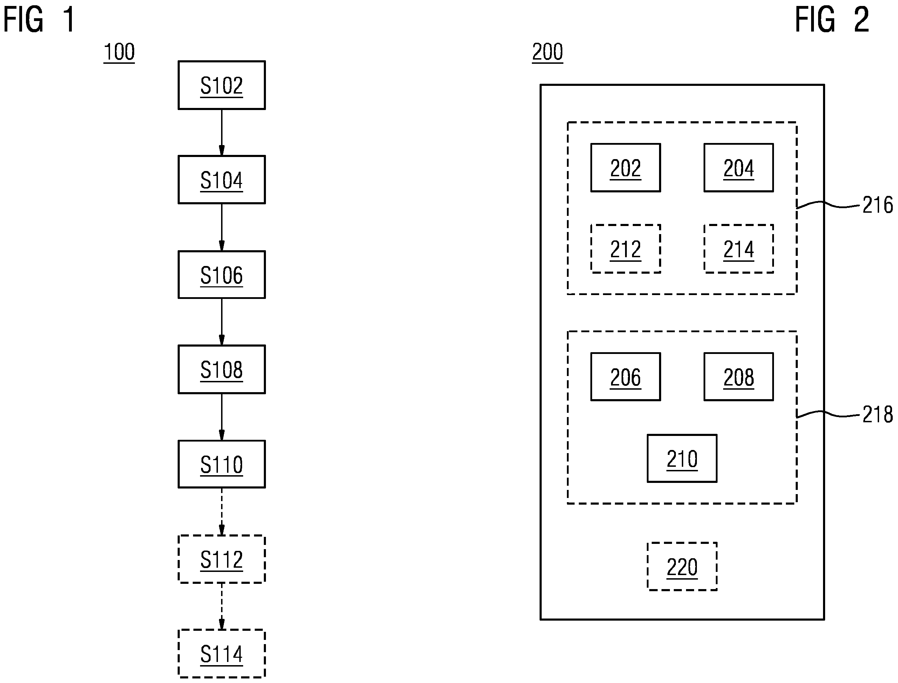

Fig. 1 schematically illustrates an exemplary flowchart for a (in particular computer-implemented) method for extracting medical imaging analysis data in relation to a medical question. The method is generally referred to by the reference sign 100.

The method 100 comprises a step S102 of receiving a first medical dataset in relation to a patient. The first medical dataset comprises first medical imaging data acquired by means of a first medical imaging modality in relation to a medical question. The method 100 further comprises a step S104 of receiving a second medical dataset in relation to the patient. The second medical dataset comprises context information in relation to the medical question. The method 100 further comprises a step S106 of converting at least part of the received S102 first medical dataset into a target format. The converting S106 of at least part of the first medical dataset into the target format may comprise constructing a 2D medical image from the first medical imaging data, in particular comprising a similar view on anatomical structures as a 2D medical image comprised in the second medical dataset.

The method 100 further comprises a step S108 of jointly analysing the at least in part converted S106 first medical dataset and the received S104 second medical dataset in view of the medical question.

The method 100 still further comprises a step S110 of extracting medical imaging analysis data in relation to the medical question from the result of the jointly analysing S108.

The method 100 may further comprise a step of providing S112 the extracted S110 medical imaging analysis data to a medical expert, in particular by using a user interface (UI), such as a graphical user interface (GUI).

Optionally, the method 100 comprises a step S114 of receiving feedback by the medical expert. The feedback may comprise an acceptance, a rejection, and/or a rating or assessment (e.g., in view of a quality, and/or by a medical practitioner, in particular a radiologist) of the medical imaging analysis data.

Fig. 2 schematically illustrates an exemplary architecture of a computing device for extracting medical imaging analysis data in relation to a medical question. The computing device is generally referred to by the reference sign 200.

The computing device 200 comprises a first interface 202, which is configured for receiving a first medical dataset in relation to a patient. The first medical dataset comprises first medical imaging data acquired by means of a first medical imaging modality in relation to a medical question. The computing device 200 further comprises a second interface 204, which is configured for receiving a second medical dataset in relation to the patient. The second medical dataset comprises context information in relation to the medical question. The computing device 200 further comprises a conversion module 206, which is configured for converting at least part of the received first medical dataset into a target format. The computing device 200 further comprises an analysis module 208, which is configured for jointly analysing the at least in part converted first medical dataset and the received second medical dataset in view of the medical question. The computing device 200 still further comprises an extraction module 210, which is configured for extracting medical imaging analysis data in relation to the medical question from the result of the jointly analysing.

The computing device 200 may comprise a providing module (not shown) and/or a third interface 212, which is configured for providing the extracted medical imaging analysis data to a medical expert. The extracted medical imaging analysis data may in particular be provided on a UI, an preferably on a GUI.

The computing device 200 may further comprise a fourth interface 214, which is configured for receiving feedback by the medical expert. The feedback may comprise an acceptance, a rejection, and/or rating (and/or assessment) of the medical imaging analysis data.

The computing device 200 may comprise an input-output interface 216. Any one of the first interface 202, second interface, 204, the optional third interface 212, and/or the optional fourth interface 214 may be comprised in the input-output interface 216.

The computing device 200 may comprise a processor 218. The conversion module 206, the analysis module 208, the extraction module 210, and/or the optional providing module may be embodied by the processor 218.

The computing device 200 may further comprise a memory 220, e.g., for storing computer program code for executing the method 100, and/or for storing the medical imaging analysis data and/or any intermediary result.

The computing device 200 may be configured for performing the method 100.

The inventive technique (e.g., comprising the method 100, and/or the computing device 200) can be viewed as a complementary arrangement, and/or an extension of arrangements, of conventional common data elements (CDE), such as RadElement (https://www.radelement.org/) for radiology. E.g., a unified platform, on which (and/or by accessing of which) the method 100 is performed, may be denoted as RadElement+. Alternatively or in addition, the technique can comprise methods for building a unified platform and/or a modality agnostic foundational framework for enabling Al to process information for prior comparison.

RadElement is an example of a platform, which offers a catalogue of radiology CDEs, indexed by title and controlled terms such as SNOMED CT, LOINC, and RadLex. CDEs can be grouped into "sets" that list the CDEs used in particular applications. CDEs can be reused, and hence may belong to more than one set. The site's web service (using REST and JSON) makes it easy for automated systems to discover and apply radiology CDEs.

It is to be noted that the actual working of RadElement and the inventive technique (e.g., comprising the method 100, and/or the computing device 200) do not closely overlap. The RadElement platform is designed to standardize radiology reporting through the use of Common Data Elements, CDEs, that can provide a uniform platform of communication between healthcare professionals (radiologists and/or referring physicians, for instance). Also, depending upon the role, information in the RadElement platform is coming from different healthcare professionals to complete the healthcare picture of an individual.

The inventive technique (e.g., comprising the method 100, and/or the computing device 200), even though termed RadElement+ by the inventors, is not simply an extension of the existing RadElement platform, which is designed for different healthcare professionals communicating to each other, with extra features. The inventive technique rather provides a similar spirited, and/or complementary, unified platform for Al instead of healthcare professionals. According to the inventive technique, different (in particular "plug and play"-style) Als designed and fine-tuned for different tasks can communicate and/or interact, e.g., to confirm or reject individual medical analyses.

The inventive technique (e.g., comprising the method 100, and/or the computing device 200) facilitates producing a unified framework (e.g., complementary to, similar to, and/or in extension of, RadElement) for information extracted by Al systems from different multimodal sources (such as CT, XR, MRT, US, and/or clinical reports, also: medical reports). This can make the framework agnostic to the Al used to accomplish different tasks (e.g., performing a segmentation on medical imaging data comprised in a, e.g., the first and/or second, medical dataset), and/or to the modality of how the prior information (such as the context information and/or the second medical dataset) is delivered.

Individual Al systems demonstrate state-of-the-art performance for highly specialized tasks (e.g., segmentation and/or detection from a specific modality, and/or text understanding through Large Language Models, LLMs), but conventionally struggle to perform more than one task, a more generic task, and/or the task once the scope of the task is broadened. The inventive technique (e.g., comprising the method 100, and/or the computing device 200) allows for a plug- and-play situation with state-of-the-art Al available for the task that can replaced once a better solution is available.

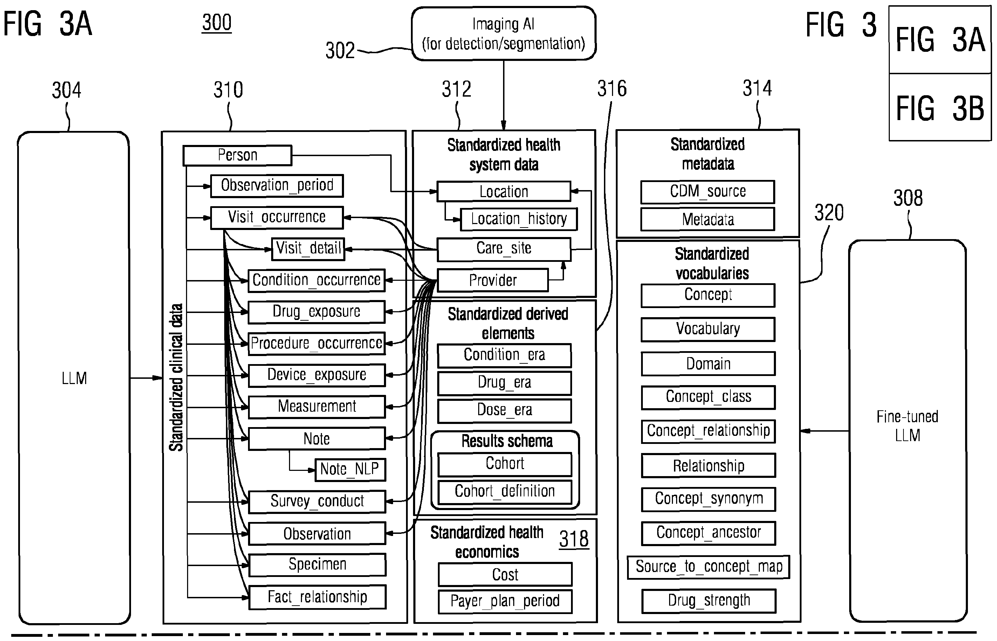

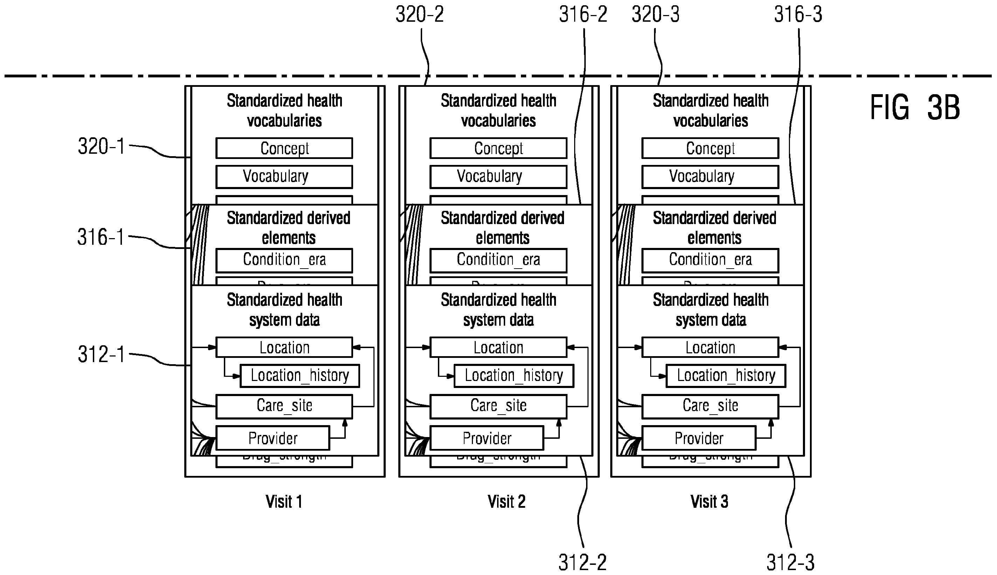

Figs. 3A and 3B shows an example of the inventive technique (also: the framework). The framework is agnostic to how and using which technology the relevant unified information at each time-point is filled up. For instance, if at different points in time (also: time points, TP), a chest X-ray is performed to monitor a nodule during visit 1 (or TP1) and CT during visit 2 (or TP2), separate X-ray and CT Al systems can be used to process those cases (e.g., performing segmentations on the medical imaging data comprised in the second and first medical data set, respectively) and extract the relevant information that can be subsequently used to perform the jointly analyzing (and/or the comparison). Similarly, if only clinical reports are available for the prior exam (e.g., the patient was transferred from another institution, and/or comprised as context information in the second medical dataset), fine-tuned LLMs can be used to extract similar (or the same) information from clinical reports that imaging Al extracted from the current exam (and/or the first medical dataset).

In Fig. 3A, exemplarily, an LLM 304, a fine-tuned LLM 306 and imaging Al 302 (e.g., for performing segmentation and/or detection, such as organ detection) are exemplarily shown for populating a unified platform 300. The unified platform 300 comprises a plurality of fields, which can be arranged in different categories, such as (e.g., standardized) clinical data 310, (e.g., standardized) health data 312, (e.g., standardized) derived elements 316, (e.g., standardized) health economics 318, (e.g., standardized) metadata 314, and/or (e.g., standardized) vocabularies 320.

In Fig. 3B, an example of populating the unified platform 300 at three different time points (in particular denoted as Visit 1, Visit 2, Visit 3) is shown. The corresponding categories populated per time point X (and/or Visit X; with X=1,2,3) are exemplarily denoted as (e.g., standardized) health data 312-X, (e.g., standardized) derived elements 316-X, and (e.g., standardized) vocabularies 320-X.

The (e.g., standardized) metadata 314 may comprise, e.g., CDM_source (with CDM for Common Data Model), and/or any further (e.g., conventional) metadata.

The (e.g., standardized) vocabularies 320 (e.g., used in visits 1,2 and 3 at reference sign 320-1; 320-2 and 320-3, respectively) may, e.g., comprise concept, (e.g., conventional) vocabulary, domain, Concept_class, Concept_replationship, relationship, Concept_synonym, Concept_ancestor, Source_to_concept_map, and/or Drug_strength.

The (e.g., standardized) health economics 318 may, e.g., comprise cost and/or payer_plan_period.

The (e.g., standardized) derived elements 316 (e.g., used in visits 1,2 and 3 at reference sign 316-1; 316-2 and 316-3, respectively) may, e.g., comprise Condition_era, Drug_era, Dose_era, and/or a Results schema, e.g., populated by fields Cohort and/or Cohort_definition.

The (e.g., standardized) health data 312 (e.g., used in visits 1,2 and 3 at reference sign 312-1; 312-2 and 312-3, respectively) may, e.g., comprise a field Location, optionally with subfield Location_history, a field Care_site and/or Provider.

The (e.g., standardized) clinical data 310 may, e.g., comprise a field Person with subfields Observation_period and/or visit_occurrence, and/or further subfields, such as Visit_detail, Condition_occurrence, Drug_exposure, Procedure_occurrence, Device_exposure, Measurement, Note, Note_NLP (with NLP for Natural Language Processing), Survey_conduct, Observation, Specimen, and/or Fact_relationship.

For each medical examination (also: visit), entries may be generated, e.g., in the categories (e.g., standardized) vocabularies, (e.g., standardized) derived elements, and/or (e.g., standardized) health data.

Standardized herein may refer to a digital data format (e.g., a text format, a graph format, a 2D image format, and/or a 3D image format). Alternatively or in addition, standardized may refer to a type of content, such as medical expressions and/or medical relationships according to a medical ontology, such as the Systematized Nomenclature of Medicine (SNOMED), in particular SNOMED CT.

The inventive technique (e.g., comprising the method 100, and/or the computing device 200) may be used for robust incorporation of prior imaging data (e.g., comprised in the second medical dataset).

Conventional methods suffer from two major issues: (1) performance degradation due to large shift of data distributions even when the prior exam was performed using the same imaging modality, and (2) lack of instance level annotations in the target domain since the prior and current exams (e.g., furnishing the second and first medical dataset, respectively) are almost in all circumstances used for comparison purposes, e.g., if something (such as a, abnormality, lesion, and/or tumor) was present and/or absent before, if it grew and/or shrank in size. Conventional approaches mainly focus on either of these two difficulties, even though they are closely coupled in cross domain object detection. To solve this problem, inventive technique can make use of a target-perceived dual-branch distillation (TDD) network (also: TDD framework). The TDD framework takes as input a current and prior study(ies), in particular as first medical dataset and one or more second medical datasets, respectively. Current and prior studies may, may not (and/or or need not), be from the same modality, but the inventive technique (e.g., comprising the method 100, and/or the device 200; briefly also: the system or the framework) may assume that the scope of detection in all modalities is the same. In an embodiment, the system may reject the case if a certain clinical question can be answered only in one imaging modality. The comparison in this scenario would be meaningless.

In case of different modalities, the system may route the higher-dimensional image (current, e.g., comprised in the first medical dataset, or prior) to a Digital Reconstructor to produce low-resolution (or a lower resolution). In an example embodiment, the framework then integrates the detection branches of both source and target domains in a unified teacher-student learning scheme. The inventive technique can reduce domain shift and generate reliable supervision effectively. In particular, a distinct Target Proposal Perceiver between two domains may be introduced. It can adaptively enhance source detection to perceive objects in a target image, by leveraging target proposal contexts from iterative cross attention. Afterwards, a concise Dual Branch Self Distillation strategy is designed for model training, which can progressively integrate complementary object knowledge from different domains via self-distillation in two branches, e.g., according to the self-distillation described in [1,2].

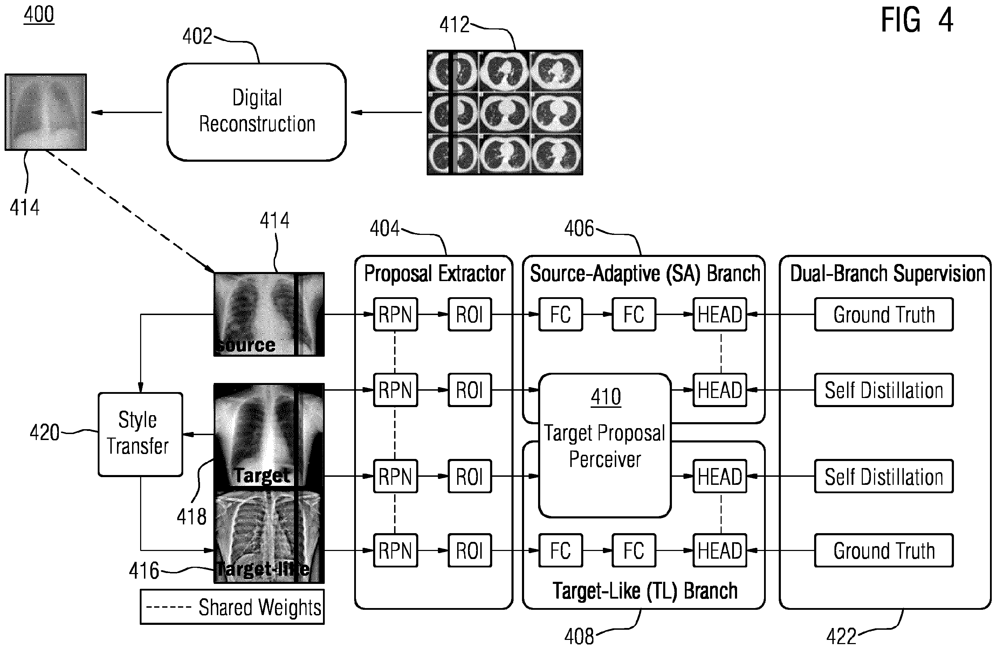

Fig. 4 schematically illustrates a TDD network 400. The TDD network 400 comprises a digital reconstructor 402. The digital reconstructor is configured for receiving and selecting from the (in particular 3D, such as comprising a CT scan) first medical imaging data 412 a similar (in particular 2D) view 414 (or transforming the first medical imaging data 412 to arrive at the similar view 414) to the view provided by (in particular 2D, such as comprising an X-ray image) second medical imaging data 418. Said differently, if the source 412 and target 418 are from different modalities (such as CT and radiography), the digital reconstructor 402 is used to reconstruct the lower-dimensional modality. A number of techniques can be used for this, for instance described in [3].

Afterwards, a source domain image 414 is transferred into target-like domain 416, and/or a style transfer 420 is performed on the view 414 of the first medical imaging data 412. The style transfer 420 is based on the style of the second medical imaging data 418, and as a result the converted first medical imaging data 416 (also denoted as target-like first medical imaging data 416) is obtained.

After the selection of the corresponding view 414 and/or the dimensional reduction of the first medical imaging data 412 is performed, parts of the TDD network 400 starting from the style transfer 420 may operate analogously to the TDD used for transferring weather and/or lighting-related domains of 2D camera images of traffic scenes described in [1].

The TDD network 400 further comprises a (in particular shared) proposal extractor 404 into which all of the images 414; 416; 418 from the three (or two, if target and target-like are counted as one) domains are fed to get proposals and proposal features. The shared property may involve shared weights of a set of region proposal networks (RPN), or using the same RPN for all three input images 414; 416; 418, as indicated by the dashed lines within the proposal extractor 404. The proposal extractor 404 is configured for identifying at least one region of interest (ROI), and/or getting proposal features, in the view 414 of the first medical imaging data (also denoted as source 414), the converted 416 first medical imaging data, and the second medical imaging data 416.

The TDD network 400 further comprises a source-adaptive (SA) block (also: SA branch) 406 and a target-like (TL) block (also: TL branch) 408. The SA block 406 is configured for performing medical image data analysis on the (in particular source) view 414 of the first medical imaging data 412 and on the second medical imaging data (also: target) 418, and the TL block 408 is configured for performing medical image data analysis on the converted (also: target-like) 416 first medical imaging data, and also on the second medical imaging data 418. The TDD network 400 further comprises a target proposal perceiver 410. The target proposal perceiver 410 is configured for comparing the medical image data analysis from the SA block 406 and from the TL block 408, in particular when performed on the same second medical imaging data (also: target) 418.

The SA block 406 and the TL block 408 each comprise one or more sets of fully connected (FC) layers and one or more projection heads (HEAD). Either the same HEAD, or a HEAD with shared weights is used for both types of medical imaging data (briefly: images) input into the corresponding block, namely the source image 414 and target image 418 into the SA block 406 and the target-like image 416 and target image 418 into the TL block 404.

The proposal features of source 414 and target-like images 416 are used to train the corresponding branches 406 and 408, respectively, with supervision of ground truth, as indicated at reference sign 422. Moreover, the proposal features of the real target domain image 418 are fed into both branches 406; 408, for learning object knowledge from both source and target-like domains. As the images 418 from the target domain are not annotated, the model is optimized by self-distillation, as also indicated a reference sign 422.

The TDD network 400 is thus trained by dual-branch supervision 422, making use of the fact that the target image 418 is processed by both the SA branch 406 and the TL branch 408, and the first medical imaging data 412 are processed with the same view, but once in the source style 414 and once in the target-like style 416, by the SA branch 406 and the TL branch 408, respectively, and use is made of an annotation (e.g., a segmentation of the first medical imaging data 412) for training both the SA branch 406 and the TL branch 408.

The dual-branch self-distillation may comprise tree phases, namely a joint-domain pretraining, a cross-domain distillation (e.g., using a teacher-student approach), and a dual-teacher refinement.

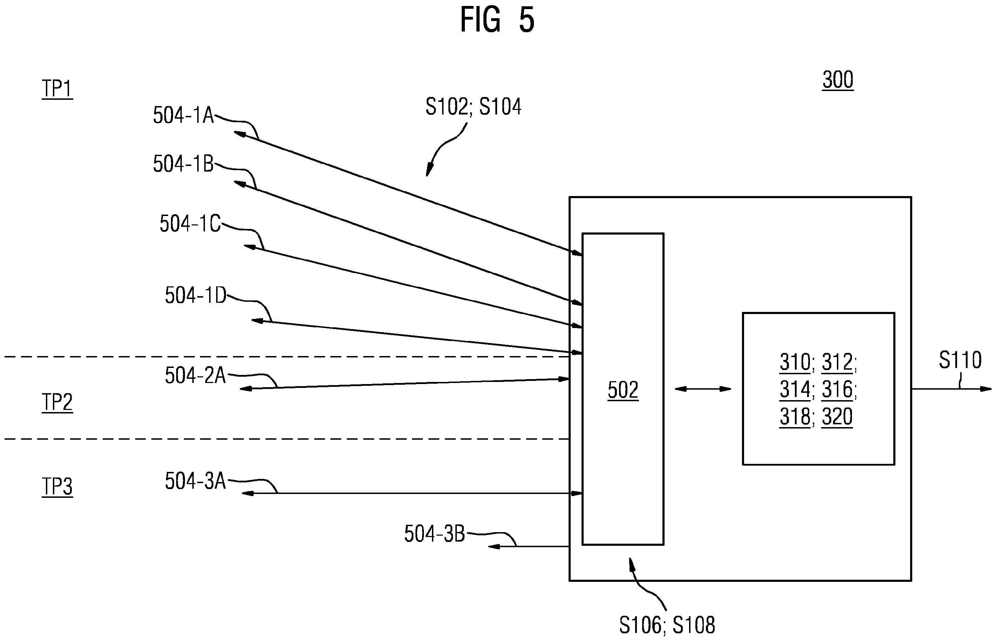

Fig. 5 shows a further example of uniform platform 300 comprising a database of extracted information, which has (e.g., standardized) fields for medical imaging findings and medical-non imaging findings (and/or clinical findings) as indicated at reference signs 310; 312; 314; 316; 318; 320. The unified platform 300 in Fig. 5 further has an Al orchestrator 502. The Al orchestrator 502 acts as manager between different Als (e.g., the Imaging Al 302, the LLM 304 and the fine-tuned LLM 308 in Fig. 3) and the database (in particular with categories of entries 310; 312; 314; 316; 318; 320). The Al orchestrator 502 is, e.g., configured to determine what (e.g., which medical imaging data and medical non-imaging data) to "listen" to (and/or submit to the database) and what to ignore (and/or not submit to the database).

In Fig. 5, exemplarily three different time points (e.g., visits) TP1; TP2; TP3 are shown, which provide different types of potential input to the database (in particular with categories of entries 310; 312; 314; 316; 318; 320). Exemplarily, at TP1 a plurality of different input data are shown, and at TP2 and TP3 exemplarily one type of input data to the database is shown. In this exemplary embodiment, each potential input is provided by an Artificial Narrow Intelligence (ANI).

In the example of Fig. 5, at TP1 different ANIs provide findings of three different chest radiographs (CRX). At reference sign 504-1A, an ANI 1a provides information on lung opacities for CXR 1, at reference sign 504-1B, an ANI 1b provides findings on lung nodules from CRX 1, at reference sign 504-1C, a consolidation using an ANI 1 and CRX 2 is performed, and at reference sign 504-1D, ANI 1 provides findings on pleural effusion using CRX 3.

At TP2 in Fig. 5, at reference sign 504-2A a CT report is provided an LLM ANI.

At TP3 in Fig. 5, a CT is analyzed using an ANI 2 at reference sign 504-3A, and at reference sign 504-3B exemplarily an output, and/or a smart report, is provided of what can already be provided (e.g., by analysing prior exams, such as at TP1 and/or TP2) to ANI 2.

Any of the individual ANIs used at reference signs 504-1A; 504-1B; 504-1C; 504-1D; 504-2A; 504-3A may be considered as "plug and play", and/or may be independently replaceable by updated ANIs and/or new ANIs, in particular in relation to the Al orchestrator 402. Alternatively or in addition, the two-way arrows between the reference signs 504-1A; 504-1B; 504-1C; 504-1D; 504-2A; 504-3A and the Al orchestrator 502 may denote (at least the possibility of) bidirectional transfer of information. E.g., an Al agent (and/or ANI) 504-1A; 504-1B; 504-1C; 504-1D; 504-2A; 504-3A can submit information to the Al orchestrator 502 for evaluation and subsequent storage. Alternatively or in addition, the Al agent (and/or ANI) 504-1A; 504-1B; 504-1C; 504-1D; 504-2A; 504-3A can request information from the Al orchestrator 502 to perform a task, such as providing (and/or obtaining) results of prior exams.

In Fig. 5, any of the CRX or CT data at TP1 and TP2; TP3, respectively, can act as first medical dataset according to the method step S102, with any of the other received findings in relation to these medical imaging data serving as candidate for the context information according to the method step S104. The Al orchestrator 502 is configured for performing the method steps S106 and S108. At reference sign S110 in Fig. 5, medical imaging analysis data in relation to the medical question are provided as output, and a report is sent to a user (e.g., a radiologist or oncologist), which may include results from ANI 2 (in particular on the CT performed at TP3) at reference sign 504-3A and/or a comparison to priors, such as based on the data 504-1A; 504-1B; 504-1C; 504-1D at TP1 and 504-2A at TP2.

The Al orchestrator 502 may be a substantial generalization of the artificial general intelligence for medical imaging analysis [5] and/or of the vision-language models of [6]. The inventive technique of the system (and/or unified platform 300) differs from prior art in particular in the modality-agnostic nature of how the information is received and stored. The Al orchestrator 502 makes this happen by coordinating between multiple (e.g., imaging) modalities and different Al agents (and/or ANIs).

The Al orchestrator 502 in Fig. 5 is trained with background knowledge of the medical domain, capable of comprehension of cause and effect-comorbidities, and is capable of abstract thinking. The Al orchestrator 502 may be trained by self-supervised learning (SSL), transfer learning, and/or reinforcement learning. Alternatively or in addition, the Al orchestrator 502 may be subject to temporal changes (e.g., using continued training and/or fine-tuning, and/or responsive to new "plug and play" ANIs providing input data), and/or may be configured to register temporal changes of data in relation to a patient. Alternatively or in addition, the Al orchestrator 502 may be configured for handling conflicting information, e.g., if two nodule Als provide different findings for the same organ, such as one Al says "normal" and the other Al says "nodule".

The unified platform 300, the Al orchestrator 502, and/or the database (in particular with categories of entries 310; 312; 314; 316; 318; 320) may be updated (e.g., periodically and/or event-based, such as if new imaging Als or new LLMs provide input data, which are of different type (e.g., different data format and/or comprising information types not previously comprised in the database).

The inventive technique (e.g., comprising the method 100, and/or the computing device 200) provides a unified platform for storing and comparing information that is currently extracted from medical images or medical images in aide with accompanying non-imaging data. The inventive technique, comprising the unified platform, is agnostic to how information was originally collected. The platform is also universally adaptable and encompasses or has the capabilities to store all the necessary information. The source of that information can vary in terms of imaging modalities sources (e.g., CT, XR, MR, US, and/or clinical reports), or Al system used.

A simple real-world example comprises storing a patient's height or weight. Once that information is stored in a form, the tool used for measuring height is not relevant. This is conventionally not the case with imaging-based Al systems: information extracted using one imaging modality, and/or one Al system, cannot conventionally be readily translated to a different modality and/or system, thus rendering the prior extracted information useless in most instances.

Since the Al systems are complex and generally good for highly specialized tasks (e.g., segmentation and/or detection from a specific modality, and/or text understanding through LLMs), creating a universal Al for comprehensive diagnostics becomes complicated. The inventive technique (e.g., comprising the method 100, and/or the computing device 200) supplies a unified platform for different Als to submit information that they are good at and/or designed for. The inventive technique lets other Al systems consume that information from that unified platform. The inventive technique thus provides a unified (also: agnostic universal) platform, in particular extending conventional platforms, such as the RadElement-like framework, and/or providing a unified communal imaging space.

The universal applicability of the inventive technique guarantees that any Al-network capable of extracting useful information from medical images can be used.

The inventive technique improves on accuracy. Conventional solutions focus solely on intensity harmonization of the source and target domains without quantifying the underlying primary task, such as detection (e.g., of health-related information in view of a specific medical question).

The inventive technique improved on reliability. The lack of performance in the source domain result in no or wrong detection can renders the entire system of prior comparison not useable, e.g., an abnormality detected as "nodule" in one domain and "pneumonia" in the other can make comparing with prior exams difficult. The inventive technique can alert a medical expert (e.g., radiologist) to this issue.

The inventive technique improves in terms of ubiquitousness. Conventional approaches assume the current and prior exams to be in the same domain and/or modality (such as chest radiography, CXR, CT). This is a conventional major bottleneck in the clinical workflow, where exams are performed using different modalities depending upon the need, institutional preferences, etc. The proposed framework is capable of working across modalities.

Independent of grammatical term usage, individuals with male, female or other gender identities are included within the term.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

### Cited Prior Art

[1] He, M., Wang, Y., Wu, J., Wang, Y., Li, H., Li, B., ... & Qiao, Y. (2022). Cross domain object detection by target-perceived dual branch distillation. In Proceedings of the IEEE/CVF Conference on Computer Vision and Pattern Recognition (pp. 9570-9580).
[2] Liang, S., Wang, W., Chen, R., Liu, A., Wu, B., Chang, E. C., ... & Tao, D. (2024). Object Detectors in the Open Environment: Challenges, Solutions, and Outlook. arXiv preprint arXiv:2403.16271.
[3] Pyrros, A., Chen, A., Rodríguez-Fernández, J. M., Borstelmann, S. M., Cole, P. A., Horowitz, J., ... & Koyejo, S. (2023). Deep Learning-Based Digitally Reconstructed Tomography of the Chest in the Evaluation of Solitary Pulmonary Nodules: A Feasibility Study. Academic radiology, 30(4), 739-748.
[4] Hong-You Chen and Zhengfeng Lai and Haotian Zhang and Xinze Wang and Marcin Eichner and Keen You and Meng Cao and Bowen Zhang and Yinfei Yang and Zhe Gan. ontrastive Localized Language-Image Pre-Training. arXiv:2410.02746 [cs.CV]
[5] Li, X., Zhao, L., Zhang, L., Wu, Z., Liu, Z., Jiang, H., ... & Shen, D. (2024). Artificial general intelligence for medical imaging analysis. IEEE Reviews in Biomedical Engineering.
[6] Zhang, J., Huang, J., Jin, S., & Lu, S. (2024). Vision-language models for vision tasks: A survey. IEEE Transactions on Pattern Analysis and Machine Intelligence.

## Claims

1. Computer-implemented method (100) for extracting medical imaging analysis data in relation to a medical question, comprising the method steps of:
- Receiving (S102) a first medical dataset in relation to a patient, wherein the first medical dataset comprises first medical imaging data acquired by means of a first medical imaging modality in relation to a medical question;
- Receiving (S104) a second medical dataset in relation to the patient, wherein the second medical dataset comprises context information in relation to the medical question;
- Converting (S106) at least part of the received (S102) first medical dataset into a target format;
- Jointly analysing (S108) the at least in part converted (S106) first medical dataset and the received (S104) second medical dataset in view of the medical question; and
- Extracting (S110) medical imaging analysis data in relation to the medical question from the result of the jointly analysing (S108).

2. Method (100) according to claim 1, wherein the received (S102) first medical dataset or the converted (S106) first medical dataset comprises a result of a perception task, optionally
wherein the perception task comprises an organ detection and/or a semantic segmentation.

3. Method (100) according to any one of the preceding claims, wherein the converting (S106) of at least part of the first medical dataset depends on the received (S104) context information and/or on the medical question.

4. Method (100) according to any one of the preceding claims, wherein the received (S104) second medical dataset comprises text data, optionally wherein the text data comprise:
• a medical report of a medical examination;
• the patient's medical history;
• the patient's medication;
• laboratory result data; and/or
• annotations in relation to medical imaging data acquired by means of a second medical imaging modality.

5. Method (100) according to any one of the preceding claims, wherein the received (S104) second medical dataset comprises second medical imaging data acquired by means of a second medical imaging modality in relation to the medical question.

6. Method (100) according to any one of the preceding claims, wherein the first medical imaging modality, and optionally the second medical imaging modality, comprises at least one of:
- Magnetic resonance tomography, MRT;
- Computed tomography, CT;
- Single-photon emission CT, SPECT;
- Positron emission tomography, PET;
- Scintigraphy;
- Radiography;
- Ultrasound, US;
- Elastography;
- Photoacoustic imaging;
- Functional near-infrared spectroscopy, FNIR: and
- Magnetic particle imaging, MPI.

7. Method (100) according to the claim 5 and 6, wherein the second medical imaging modality differs from the first medical imaging modality, and wherein the second medical imaging data was acquired prior to the first medical imaging data.

8. Method (100) according to any one of claims 5 to 7, wherein the second medical imaging data are lower dimensional than the first medical imaging data, in particular
wherein the second medical imaging data comprise a two-dimensional, 2D, medical image, and wherein the first medical imaging data comprise a three-dimensional, 3D, medical image; and/or
wherein converting (S106) at least part of the first medical dataset into the target format comprises constructing a 2D medical image from the first medical imaging data, in particular comprising a similar view on anatomical structures as the 2D medical image comprised in the second medical dataset.

9. Method (100) according to any one of claims 5 to 8, wherein the method (100) is performed by a target-perceived dual-branch distillation, TDD, network (400), wherein the TDD network (400) comprises:
- A digital reconstructor (402) configured for converting (S106) the first medical imaging data and obtaining a similar view of the view provided by the second medical imaging data;
- A proposal extractor (404), which is configured for identifying at least one region of interest, ROI, in the converted first medical imaging data and the second medical imaging data;
- A source-adaptive, SA, block (406) and a target-like, TL, block (408), wherein the SA block (406) is configured for performing medical image data analysis on the received (S102) first medical imaging data, and wherein the TL block (408) is configured for performing medical image data analysis on the converted (S106) first medical imaging data, and optionally on second medical imaging data comprised in the second medical dataset; and
- a target proposal perceiver (410) configured for comparing the medical image data analysis from the SA block (406) and from the TL block (408).

10. Method (100) according to any one of the preceding claims, wherein the step of jointly analyzing (S108) the at least in part converted first medical dataset and the received second medical dataset, and/or the extracting (S110) of the medical imaging analysis dataset is stopped if it is determined that the second medical dataset cannot answer the medical question associated with the first medical dataset.

11. Method (100) according to any one of the preceding claims, further comprising a step of providing (S112) the extracted (S110) medical imaging analysis data to a medical expert, in particular by using a graphical user interface, GUI; optionally
the method (100) further comprising receiving (S114) feedback by the medical expert, wherein the feedback comprises at least one of:
• An acceptance;
• A rejection; and
• A rating or assessment
of the medical imaging analysis data.

12. Unified platform (300) for extracting medical imaging analysis data in relation to a medical question, wherein the unified platform is configured for storing a plurality of, in particular second, medical datasets, and wherein the unified platform is further configured to be accessed for performing the method (100) according to any one of the preceding method claims.

13. Computing device (200) for extracting medical imaging analysis data in relation to a medical question, comprising:
- A first interface (202) configured for receiving a first medical dataset in relation to a patient, wherein the first medical dataset comprises first medical imaging data acquired by means of a first medical imaging modality in relation to a medical question;
- A second interface (204) configured for receiving a second medical dataset in relation to the patient, wherein the second medical dataset comprises context information in relation to the medical question;
- A conversion module (206) configured for converting at least part of the received first medical dataset into a target format;
- An analysis module (208) configured for jointly analysing the at least in part converted first medical dataset and the received second medical dataset in view of the medical question; and
- An extraction module (210) configured for extracting medical imaging analysis data in relation to the medical question from the result of the jointly analysing.

14. Computing device (200) according to the directly preceding claim, further configured to perform any one of the steps, and/or comprise any one of the features, of any one of the method claims 2 to 11.

15. A computer program product comprising program elements which induce a computing device (200) to carry out the steps of the method for extracting medical imaging analysis data in relation to a medical question according to any one of the preceding method claims, when the program elements are loaded into a memory of the computing device (200).

## Amended claims

### Amended claims in accordance with Rule 137(2) EPC.

1. Computer-implemented method (100) for extracting medical imaging analysis data in relation to a medical question, comprising the method steps of:
- Receiving (S102) a first medical dataset in relation to a patient, wherein the first medical dataset comprises first medical imaging data (412) acquired by means of a first medical imaging modality in relation to a medical question;
- Receiving (S104) a second medical dataset in relation to the patient, wherein the second medical dataset comprises context information in relation to the medical question;
- Converting (S106) at least part of the received (S102) first medical dataset into a target format, the target format being a format in a shared space, in which both the received (S102) first medical dataset and the received (S104) second medical dataset are processable;
- Jointly analysing (S108) the at least in part converted (S106) first medical dataset and the received (S104) second medical dataset in view of the medical question; and
- Extracting (S110) medical imaging analysis data in relation to the medical question from the result of the jointly analysing (S108), wherein the extracted (S110) medical imaging analysis data comprises at least one of a non-existence of an anomaly, an existence of an anomaly, a type of an anomaly, and a progression of an anomaly.

2. Method (100) according to claim 1, wherein the received (S102) first medical dataset or the converted (S106) first medical dataset comprises a result of a perception task, optionally
wherein the perception task comprises an organ detection and/or a semantic segmentation.

3. Method (100) according to any one of the preceding claims, wherein the converting (S106) of at least part of the first medical dataset depends on the received (S104) context information and/or on the medical question.

4. Method (100) according to any one of the preceding claims, wherein the received (S104) second medical dataset comprises text data, optionally wherein the text data comprise:
• a medical report of a medical examination;
• the patient's medical history;
• the patient's medication;
• laboratory result data; and/or
• annotations in relation to medical imaging data acquired by means of a second medical imaging modality.

5. Method (100) according to any one of the preceding claims, wherein the received (S104) second medical dataset comprises second medical imaging data (418) acquired by means of a second medical imaging modality in relation to the medical question.

6. Method (100) according to any one of the preceding claims, wherein the first medical imaging modality, and optionally the second medical imaging modality, comprises at least one of:
- Magnetic resonance tomography, MRT;
- Computed tomography, CT;
- Single-photon emission CT, SPECT;
- Positron emission tomography, PET;
- Scintigraphy;
- Radiography;
- Ultrasound, US;
- Elastography;
- Photoacoustic imaging;
- Functional near-infrared spectroscopy, FNIR: and
- Magnetic particle imaging, MPI.

7. Method (100) according to the claim 5 and 6, wherein the second medical imaging modality differs from the first medical imaging modality, and wherein the second medical imaging data (418) was acquired prior to the first medical imaging data (412).

8. Method (100) according to any one of claims 5 to 7, wherein the second medical imaging data (418) are lower dimensional than the first medical imaging data (412), in particular
wherein the second medical imaging data (418) comprise a two-dimensional, 2D, medical image, and wherein the first medical imaging data (412) comprise a three-dimensional, 3D, medical image; and/or
wherein converting (S106) at least part of the first medical dataset into the target format comprises constructing a 2D medical image from the first medical imaging data (412), in particular comprising a similar view (414) on anatomical structures as the 2D medical image comprised in the second medical dataset.

9. Method (100) according to any one of claims 5 to 8, wherein the method (100) is performed by a target-perceived dual-branch distillation, TDD, network (400), wherein the TDD network (400) comprises:
- A digital reconstructor (402) configured for converting (S106) the first medical imaging data (412) and obtaining a similar view (414) of the view provided by the second medical imaging data (418);
- A proposal extractor (404), which is configured for identifying at least one region of interest, ROI, in the converted (S106) first medical imaging data (416) and the second medical imaging data (418);
- A source-adaptive, SA, block (406) and a target-like, TL, block (408), wherein the SA block (406) is configured for performing medical image data analysis on the received (S102) first medical imaging data (412), and wherein the TL block (408) is configured for performing medical image data analysis on the converted (S106) first medical imaging data (416), and optionally on second medical imaging data (418) comprised in the second medical dataset; and
- a target proposal perceiver (410) configured for comparing the medical image data analysis from the SA block (406) and from the TL block (408).

10. Method (100) according to any one of the preceding claims, wherein the step of jointly analyzing (S108) the at least in part converted first medical dataset and the received second medical dataset, and/or the extracting (S110) of the medical imaging analysis dataset is stopped if it is determined that the second medical dataset cannot answer the medical question associated with the first medical dataset.

11. Method (100) according to any one of the preceding claims, further comprising a step of providing (S112) the extracted (S110) medical imaging analysis data to a medical expert, in particular by using a graphical user interface, GUI; optionally
the method (100) further comprising receiving (S114) feedback by the medical expert, wherein the feedback comprises at least one of:
• An acceptance;
• A rejection; and
• A rating or assessment
of the medical imaging analysis data.

12. Unified platform (300) for extracting medical imaging analysis data in relation to a medical question, wherein the unified platform is configured for storing a plurality of, in particular second, medical datasets, and wherein the unified platform is further configured to be accessed for performing the method (100) according to any one of the preceding method claims.

13. Computing device (200) for extracting medical imaging analysis data in relation to a medical question, comprising:
- A first interface (202) configured for receiving a first medical dataset in relation to a patient, wherein the first medical dataset comprises first medical imaging data (412) acquired by means of a first medical imaging modality in relation to a medical question;
- A second interface (204) configured for receiving a second medical dataset in relation to the patient, wherein the second medical dataset comprises context information in relation to the medical question;
- A conversion module (206) configured for converting at least part of the received first medical dataset into a target format, the target format being a format in a shared space, in which both the received first medical dataset and the received second medical dataset are processable;
- An analysis module (208) configured for jointly analysing the at least in part converted first medical dataset and the received second medical dataset in view of the medical question; and
- An extraction module (210) configured for extracting medical imaging analysis data in relation to the medical question from the result of the jointly analysing, wherein the extracted medical imaging analysis data comprises at least one of a non-existence of an anomaly, an existence of an anomaly, a type of an anomaly, and a progression of an anomaly.

14. Computing device (200) according to the directly preceding claim, further configured to perform any one of the steps, and/or comprise any one of the features, of any one of the method claims 2 to 11.

15. A computer program product comprising program elements which induce a computing device (200) to carry out the steps of the method for extracting medical imaging analysis data in relation to a medical question according to any one of the preceding method claims, when the program elements are loaded into a memory of the computing device (200).
